# EUROPEAN PATENT APPLICATION

(11) **EP 4 523 634 A1**
(43) Date of publication of application: **19.03.2025**
(21) Application number: 23802441.8
(22) Date of filing: 15.02.2023
(51) Int. Cl.: A61B 17/17, A61B 34/00, A61B 8/08, A61B 17/32, A61B 34/30

(54) **METHOD AND SYSTEM FOR JUDGING POSITION OF ULTRASONIC OSTEOTOME**

(30) Priority: 10.05.2022 CN 202210507538
(71) Applicant: SMTP Medical Co., Ltd., Beijing 102629 (CN)
(72) Inventor: CAO, Qun, Beijing 102629 (CN); ZHAN, Songtao, Beijing 102629 (CN); DAI, Zhiling, Beijing 102629 (CN)
(74) Representative: Laqua, Bernd Christian Kurt
(86) International application number: PCT/CN2023/076181
(87) International publication number: WO 2023/216665

(57) **Abstract**

Disclosed are a method for determining a position of an ultrasonic osteotome (10), a system, a computer-readable storage medium, and a computer program product. The method includes: obtaining an acoustic impedance signal of the ultrasonic osteotome (10); and determining a current position of the ultrasonic osteotome (10) in bone tissue to be cut based on the acoustic impedance signal. In this way, an operator (50) can conveniently obtain the position of the ultrasonic osteotome (10) in real time, which can effectively prevent a movement path of the ultrasonic osteotome (10) from deviating from a target path, thereby reducing damage to soft tissue during surgery.

## Description

### TECHNICAL FIELD

The present application relates to the technical field of medical instruments, and in particular, to a method for determining a position of an ultrasonic osteotome, a system, a computer-readable storage medium, and a computer program product.

### BACKGROUND ART

Ultrasonic osteotomes have been widely used for cutting and breaking bone tissue. Although the ultrasonic osteotomes have the property of sparing soft tissue, they may still cause damage to the soft tissue when applying certain pressure to the soft tissue. Especially during spinal surgery or neurosurgery, if further cutting down is not stopped after the inner cortical bone has been cut, the ultrasonic osteotome may still cause damage to nervous tissue such as the spinal cord or the brain in bone cavities. Therefore, how to reduce the damage to the soft tissue during surgery is of great significance.

### SUMMARY OF THE INVENTION

The present application is intended to solve at least one of the technical problems in the related art. To this end, an objective of the present application is to provide a method for determining a position of an ultrasonic osteotome, a system, a computer-readable storage medium, and a computer program product, so as to reduce damage to soft tissue during surgery.

According to an embodiment of a first aspect of the present application, there is provided a method for determining a position of an ultrasonic osteotome, including: obtaining an acoustic impedance signal of the ultrasonic osteotome; and determining a current position of the ultrasonic osteotome in bone tissue to be cut based on the acoustic impedance signal.

In some embodiments, the determining a current position of the ultrasonic osteotome in bone tissue to be cut based on the acoustic impedance signal includes: determining that the current position is an interface between other tissue and cortical bone in response to determining that the acoustic impedance signal is on a first rising edge in a preset time and an average slope of the first rising edge is within a first slope range.

In some embodiments, the determining that the current position is an interface between other tissue and cortical bone includes: determining that the current position is an interface between air and the cortical bone in response to determining that an acoustic impedance amplitude at a start point of the first rising edge is within a first preset range.

In some embodiments, the determining that the current position is an interface between other tissue and cortical bone includes: determining that the current position is an interface between soft tissue and the cortical bone in response to determining that an acoustic impedance amplitude at a start point of the first rising edge is within a second preset range.

In some embodiments, the determining that the current position is an interface between other tissue and cortical bone includes: determining that the current position is an interface between cancellous bone and the cortical bone in response to determining that an acoustic impedance amplitude at a start point of the first rising edge is within a third preset range.

In some embodiments, the determining a current position of the ultrasonic osteotome in bone tissue to be cut based on the acoustic impedance signal includes: determining that the current position is an interface between cortical bone and other tissue in response to determining that the acoustic impedance signal is on a first falling edge in a preset time and an average slope of the first falling edge is within a second slope range.

In some embodiments, the determining that the current position is an interface between cortical bone and other tissue includes: determining that the current position is an interface between the cortical bone and cancellous bone in response to determining that an acoustic impedance amplitude at an end point of the first falling edge is within a third preset range.

In some embodiments, the determining that the current position is an interface between cortical bone and other tissue includes: determining that the current position is an interface between the cortical bone and soft tissue in response to determining that an acoustic impedance amplitude at an end point of the first falling edge is within a second preset range.

In some embodiments, the determining that the current position is an interface between cortical bone and other tissue includes: determining that the current position is in the cortical bone in response to determining that the acoustic impedance signal is consistently within a fourth preset range throughout a preset time.

In some embodiments, the determining that the current position is an interface between cortical bone and other tissue includes: determining that the current position is in the cancellous bone in response to determining that the acoustic impedance signal is consistently within a third preset range throughout a preset time.

In some embodiments, the bone tissue to be cut includes outer cortical bone, the cancellous bone, and inner cortical bone arranged sequentially from outside to inside. The method further includes: sending first prompt information in response to determining that the current position is an interface between the inner cortical bone and the soft tissue.

In some embodiments, the determining that the current position is an interface between the inner cortical bone and the soft tissue includes: determining that the current position is the interface between the inner cortical bone and the soft tissue in response to determining that the current position is at the interface between the cortical bone and the other tissue for the second time.

In some embodiments, the bone tissue to be cut includes the cortical bone and the cancellous bone arranged sequentially from outside to inside. The method further includes: sending second prompt information in response to determining that displacement of the ultrasonic osteotome is within a preset size and the current position is the interface between the cancellous bone and the cortical bone

In some embodiments, the bone tissue to be cut includes the cortical bone. The method further includes: sending third prompt information in response to determining that the current position is the interface between the cortical bone and the soft tissue.

According to an embodiment of a second aspect of the present application, there is provided an ultrasonic surgical system, including: an ultrasonic osteotome configured to cut bone tissue to be cut; and a processor configured to perform the method described above.

In some embodiments, the ultrasonic surgical system further includes: a surgical robot connected to the ultrasonic osteotome; and a display device configured to display an acoustic impedance signal of the ultrasonic osteotome. The processor is further configured to operate the ultrasonic osteotome by controlling the surgical robot.

According to an embodiment of a third aspect of the present application, there is provided a non-transitory computer-readable storage medium storing computer instructions, where the computer instructions are used to cause a computer to perform the method described in any of the above.

According to an embodiment of a fourth aspect of the present application, there is provided a computer program product, including a computer program, where the computer program, when executed by a processor, causes the method described in any of the above to be implemented.

According to the method for determining a position of an ultrasonic osteotome, the system, the computer-readable storage medium, and the computer program product provided in the embodiments of the present application, the current position of the ultrasonic osteotome in the bone tissue to be cut is determined based on the acoustic impedance signal, so that an operator can conveniently obtain the position of the ultrasonic osteotome in real time, which can effectively prevent a movement path of the ultrasonic osteotome from deviating from a target path, thereby reducing damage to soft tissue during surgery.

### BRIEF DESCRIPTION OF THE DRAWINGS

In the accompanying drawings, the same reference signs denote the same or similar components or elements throughout a plurality of accompanying drawings unless otherwise specified. These accompanying drawings are not necessarily drawn to scale. It should be understood that these accompanying drawings depict only some embodiments disclosed according to the present application and are not to be construed as limiting the scope of the present application.
FIG. 1A is a flowchart of a method for determining a position of an ultrasonic osteotome according to some embodiments of the present application;
FIG. 1B is a diagram of a working principle of an ultrasonic surgical system according to some embodiments of the present application;
FIG. 2 shows an implementation of step S102 in FIG. 1;
FIG. 3A is a diagram of a working principle of an ultrasonic surgical system according to some embodiments of the present application;
FIG. 3B is a graph of acoustic impedance variations of an ultrasonic osteotome in FIG. 3A;
FIG. 3C is a flowchart of a method for determining a position of an ultrasonic osteotome applied to the ultrasonic surgical system of FIG. 3A;
FIG. 4A is a diagram of a working principle of an ultrasonic surgical system according to some embodiments of the present application;
FIG. 4B is a graph of acoustic impedance variations of an ultrasonic osteotome in FIG. 4A;
FIG. 4C is a flowchart of a method for determining a position of an ultrasonic osteotome applied to the ultrasonic surgical system of FIG. 4A;
FIG. 5A is a diagram of a working principle of an ultrasonic surgical system according to some embodiments of the present application;
FIG. 5B is a graph of acoustic impedance variations of an ultrasonic osteotome in FIG. 5A;
FIG. 5C is a flowchart of a method for determining a position of an ultrasonic osteotome applied to the ultrasonic surgical system of FIG. 5A;
FIG. 6 is a diagram of a structure of an ultrasonic surgical system according to some embodiments of the present application; and
FIG. 7 is a partial enlarged view of an ultrasonic osteotome in FIG. 6.

### List of reference signs:

| | | | |
|---|---|---|---|
| 10: | ultrasonic osteotome; | 11: | transducer; |
| 20: | bone tissue to be cut; | 21: | cortical bone; |
| 21a: | outer cortical bone; | 21b: | inner cortical bone; |
| 22: | cancellous bone; | 30: | processor; |
| 40: | display device; | 50: | operator; |
| 51: | surgical robot; | 60: | ultrasonic driving power supply. |

### DETAILED DESCRIPTION OF EMBODIMENTS

Only some exemplary embodiments are briefly described below. As can be appreciated by those skilled in the art, the described embodiments can be modified in various ways without departing from the spirit or scope of the present application. Accordingly, the accompanying drawings and the description are considered as illustrative in nature, rather than limiting.

In the related art, during ultrasonic surgery, a cutting position is usually detected in an image positioning manner. However, due to respiratory movement of a patient and bone position deformation during a cutting process, there may be positioning errors in the image positioning manner, which is prone to damaging soft tissue. Especially during spinal surgery or neurosurgery, the soft tissue is usually nervous tissue such as the spinal cord or the brain in bone cavities, and a patient will be severely affected if the soft tissue is damaged.

To solve at least one of the above problems, the present application provides a method for determining a position of an ultrasonic osteotome and a system. A current position of the ultrasonic osteotome in bone tissue to be cut is determined based on an acoustic impedance signal, so that an operator can conveniently obtain the position of the ultrasonic osteotome in real time. This has higher precision compared with image positioning, which can effectively prevent a movement path of the ultrasonic osteotome from deviating from a target path, thereby reducing damage to soft tissue during surgery.

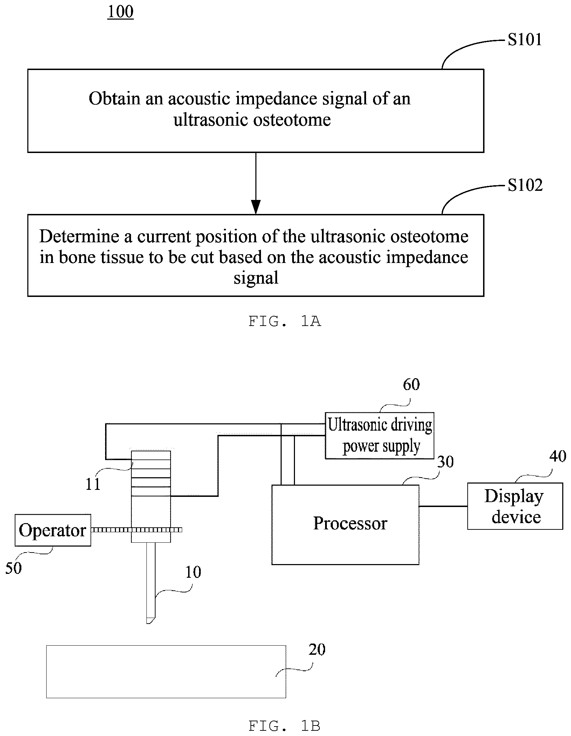

The embodiments of the present application are described below with reference to the accompanying drawings. FIG. 1A is a flowchart of a method for determining a position of an ultrasonic osteotome according to some embodiments of the present application; and FIG. 1B is a diagram of a working principle of an ultrasonic surgical system according to some embodiments of the present application. Referring to FIG. 1A and FIG. 1B, this embodiment provides a method 100 for determining a position of an ultrasonic osteotome, which may be used in open surgery or minimally invasive surgery using the ultrasonic osteotome, especially in surgeries related to bone tissue. The method 100 includes steps S101 and S102.

Step S101: Obtain an acoustic impedance signal of an ultrasonic osteotome 10.

Step S102: Determine a current position of the ultrasonic osteotome 10 in bone tissue 20 to be cut based on the acoustic impedance signal.

As shown in FIG. 1B, the ultrasonic osteotome 10 may be a common structure in the related art that can use ultrasonic energy to cut bone tissue. The ultrasonic osteotome may include a body and a transducer 11. The body has a tip that is in contact with a patient, and the tip can be used as a cutting executor. The tip may be of a variety of shapes, including various tips for cutting, grinding, drilling, etc. The transducer 11 may be connected to an ultrasonic driving power supply 60 and the tip, to convert electrical energy into ultrasonic vibrations, and may be connected to a feedback circuit that provides acquired acoustic impedance information. The ultrasonic driving power supply 60 is a power supply apparatus that may generate an ultrasonic driving signal.

The acoustic impedance signal may be used to reflect damping properties of particles at a certain position in a medium caused by a mechanical perturbation. The acoustic impedance signal may be represented as an acoustic impedance curve or a data table, etc. It may be understood that, for the ultrasonic osteotome 10, acoustic impedance varies when the tip is in contact with different media such as air, soft tissue, and bone tissue. The bone tissue may be structurally divided into cortical bone and cancellous bone, and the acoustic impedance also varies when the tip is in contact with the cortical bone and the cancellous bone. The acoustic impedance signal may be obtained in real time at a specific sampling frequency.

The bone tissue 20 to be cut is bone tissue that needs to be cut using the ultrasonic osteotome 10. There may be a plurality of types of bone tissue 20 to be cut. For example, the bone tissue to be cut may be the cortical bone, or may be a combination structure of the cortical bone and the cancellous bone, which may specifically vary depending on a type of surgery or a location of surgery.

The current position may be information about the current position of the tip of the ultrasonic osteotome 10 in the bone tissue to be cut. An operator 50 may be a doctor or a surgical robot that operates the ultrasonic osteotome 10, which is not limited.

During surgery, the acoustic impedance signal of the ultrasonic osteotome 10 is obtained in real time, and the current position of the tip of the ultrasonic osteotome 10 can be determined based on different acoustic impedance signals fed back from different types of bone tissue and their interfaces, so as to determine whether a cutting position meets surgical requirements, achieving simultaneous cutting and detection. Compared with image positioning in the related art, the method has higher precision, which can effectively prevent a movement path of the ultrasonic osteotome from deviating from a target path, thereby reducing damage to nervous tissue such as the spinal cord or the brain during surgery, improving surgical safety and surgical efficiency.

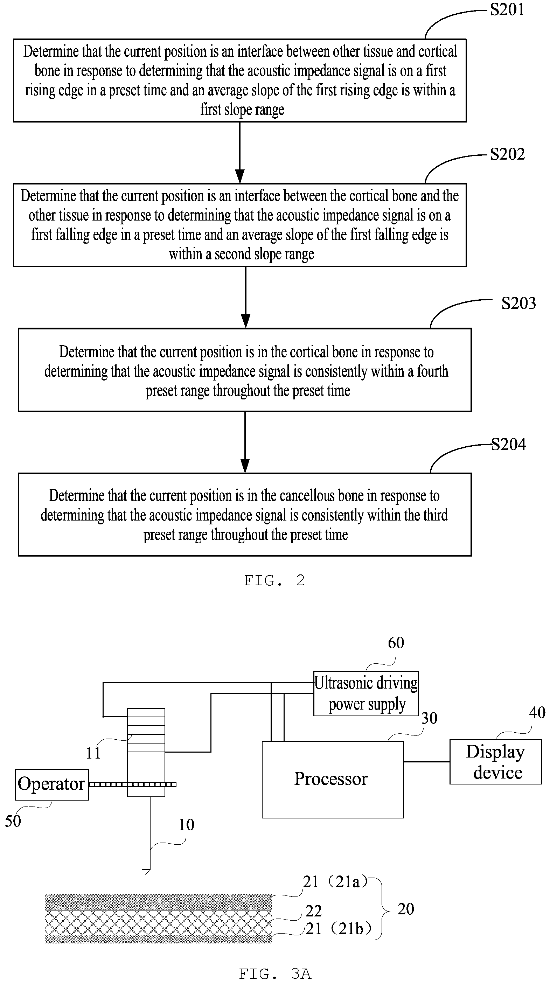

FIG. 2 shows an implementation of step S102 in FIG. 1. Referring to FIG. 2, determining the current position of the ultrasonic osteotome 10 in the bone tissue 20 to be cut based on the acoustic impedance signal may include the following step. Step S201: Determine that the current position is an interface between other tissue and cortical bone in response to determining that the acoustic impedance signal is on a first rising edge in a preset time and an average slope of the first rising edge is within a first slope range.

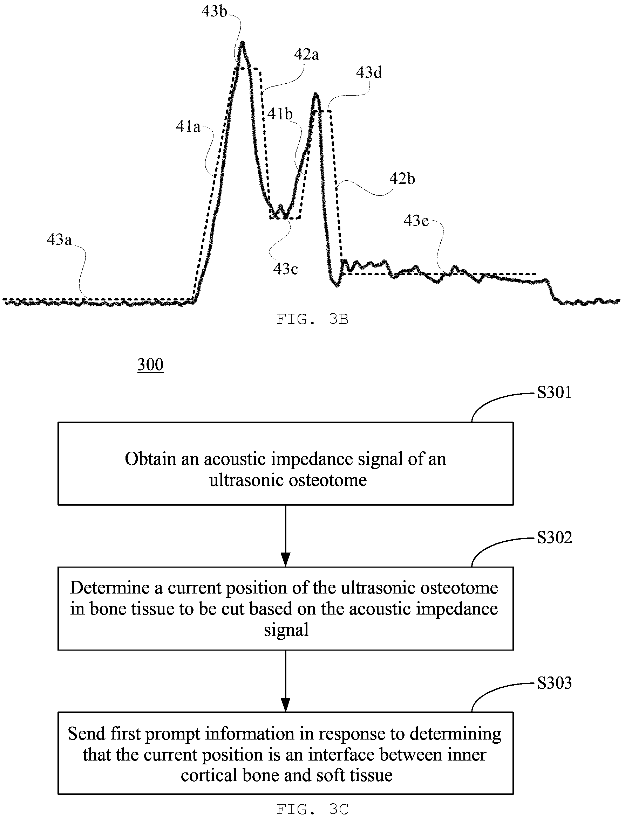

To facilitate the description of step S201, FIG. 3A is a diagram of a working principle of an ultrasonic surgical system according to some embodiments of the present application. FIG. 3B is a graph of acoustic impedance variations of an ultrasonic osteotome in FIG. 3A. Referring to FIG. 3A and FIG. 3B, in some embodiments, the bone tissue 20 to be cut includes outer cortical bone 21a, cancellous bone 22, and inner cortical bone 21b arranged sequentially from a side close to the ultrasonic osteotome 10 to a side away from the ultrasonic osteotome 10. The inner cortical bone 21b and the outer cortical bone 21a are both cortical bone 21, and inner and outer sides respectively represent position relationships between them and the cancellous bone 22. The outer side may be a position to be cut earlier during surgery, and the inner side may be a position to be cut later during surgery.

FIG. 3B shows the acoustic impedance signal, which is represented as an acoustic impedance curve, of the ultrasonic osteotome 10 cutting through the outer cortical bone 21a, the cancellous bone 22, and the inner cortical bone 21b sequentially. A horizontal coordinate axis is time, and a vertical coordinate axis is an amplitude of the acoustic impedance. In FIG. 3B, the solid line represents an acoustic impedance variation curve during experiment, and the dashed line represents an ideal acoustic impedance variation curve. It may be understood that due to the existence of interference, the acoustic impedance variation curve during the experiment has certain jitter and deviation compared with the ideal acoustic impedance variation curve.

In step S201, the first rising edge is a process in which the amplitude of the acoustic impedance signal increases with time. The dashed line in FIG. 3B is used as an example, and 41a and 41b both represent first rising edges.

The preset time may be set to a plurality of sampling periods based on actual situations. By determining whether the amplitude of the acoustic impedance signal gradually increases with time in the preset time, it may be determined whether the acoustic impedance signal is on the first rising edge. In addition, by setting the preset time appropriately, the interference of signal jitter to the determining of the first rising edge may be avoided.

A slope of the first rising edge may be an included angle between a first falling edge and the horizontal coordinate axis, and the average slope of the first rising edge is an average value of slopes of the first rising edge in the preset time. The first slope range is a numerical interval, which may be specifically set based on an actual cutting speed. For example, when a cutting speed of the ultrasonic osteotome 10 is greater than or equal to 0.5 millimeters/second (mm/s), with seconds as the unit of the horizontal coordinate axis of the acoustic impedance signal, and ohms as the unit of the vertical axis of the acoustic impedance signal, the first slope range is from 45 degrees (°) to 90 degrees, such as 45 degrees, 50 degrees, 55 degrees, 60 degrees, 65 degrees, 70 degrees, 75 degrees, 80 degrees, 85 degrees, or 90 degrees. In a preferred embodiment, the first slope range is from 60 degrees to 90 degrees, that is, the first slope range may be greater than or equal to 60 degrees and less than or equal to 90 degrees, so that the current position of the tip can be determined accurately.

The other tissue may be tissue other than the cortical bone, such as the cancellous bone, the soft tissue, or air. The interface between the other tissue and the cortical bone 21 represents that the other tissue is on the outer side of the cortical bone 21, and an incision direction of the tip is from the other tissue towards the cortical bone 21.

With reference to the first rising edges 41a and 41b in FIG. 3B, if the acoustic impedance signal is on the first rising edge and its average slope is within the first slope range, it may be accurately determined that the current position is the interface between the other tissue and the cortical bone 21, thereby facilitating the operator 50 in knowing the current position of the ultrasonic osteotome 10.

In some embodiments, determining that the current position is the interface between the other tissue and the cortical bone in step S201 may include: determining that the current position is an interface between air and the cortical bone in response to determining that an acoustic impedance amplitude at a start point of the first rising edge is within a first preset range.

It may be understood that the acoustic impedance varies when the ultrasonic osteotome 10 is in different types of tissue. Therefore, an interface between specific tissue and the cortical bone 21 which the ultrasonic osteotome 10 is at may be determined through the acoustic impedance amplitude at the start point of the entire first rising edge 41a.

The first preset range is an acoustic impedance amplitude range of the ultrasonic osteotome 10 in air. By determining whether the acoustic impedance amplitude at the start point of the first rising edge 41a is within the first preset range, it may be accurately determined whether the current position is the interface between air and the cortical bone 21.

In addition, determining that the current position is the interface between the other tissue and the cortical bone in step S201 may further include:
determining that the current position is an interface between the soft tissue and the cortical bone in response to determining that an acoustic impedance amplitude at a start point of the first rising edge is within a second preset range.

The second preset range is an acoustic impedance amplitude range of the ultrasonic osteotome 10 in the soft tissue. Similarly, by determining whether the acoustic impedance amplitude at the start point of the first rising edge 41a is within the second preset range, it may be accurately determined whether the current position is the interface between the soft tissue and the cortical bone 21.

Certainly, determining that the current position is the interface between the other tissue and the cortical bone in step S201 may further include: determining that the current position is an interface between the cancellous bone and the cortical bone in response to determining that an acoustic impedance amplitude at a start point of the first rising edge is within a third preset range.

The third preset range is an acoustic impedance amplitude range of the ultrasonic osteotome 10 in the cancellous bone. Similarly, by determining whether the acoustic impedance amplitude at the start point of the first rising edge 41b is within the third preset range, it may be accurately determined whether the current position is the interface between the cancellous bone 22 and the cortical bone 21.

In addition, the minimum value of the third preset range is greater than the maximum value of the first preset range, and the minimum value of the third preset range is greater than the maximum value of the second preset range.

It may be understood that for the cutting of three-layer bone tissue, when the cutting speed of the ultrasonic osteotome 10 is greater than or equal to 0.5 mm/s, the average slope of the first rising edge 41a of the interface between air or the soft tissue and the outer cortical bone 21a ranges 45 degrees to 90 degrees, and the average slope of the first rising edge 41b of the interface between the cancellous bone 22 and the inner cortical bone 21b ranges 45 degrees to 90 degrees.

As shown in FIG. 2, in some embodiments, determining the current position of the ultrasonic osteotome 10 in the bone tissue 20 to be cut based on the acoustic impedance signal in step S102 may further include the following step. Step S202: Determine that the current position is an interface between the cortical bone and the other tissue in response to determining that the acoustic impedance signal is on a first falling edge in a preset time and an average slope of the first falling edge is within a second slope range.

Still referring to FIG. 3B, in step S202, the first falling edge is a process in which the amplitude of the acoustic impedance signal decreases with time. The dashed line in FIG. 3B is used as an example, and 42a and 42b both represent first falling edges.

The preset time may be set to a plurality of sampling periods based on actual situations. By determining whether the amplitude of the acoustic impedance signal gradually decreases with time in the preset time, it may be determined whether the acoustic impedance signal is on the first falling edge. In addition, by setting the preset time appropriately, the interference of signal jitter to the determining of the first falling edge may be avoided.

A slope of the first falling edge may be an included angle between the first falling edge and the horizontal coordinate axis, and the average slope of the first falling edge is an average value of slopes of the first falling edge in the preset time. The second slope range is a numerical interval, which may be specifically set based on an actual cutting speed. For example, when the cutting speed of the ultrasonic osteotome 10 is greater than or equal to 0.5 millimeters/second, with seconds as the unit of the horizontal coordinate axis of the acoustic impedance signal, and ohms as the unit of the vertical axis of the acoustic impedance signal, the second slope range is from 45 degrees to 90 degrees, such as 45 degrees, 50 degrees, 55 degrees, 60 degrees, 65 degrees, 70 degrees, 75 degrees, 80 degrees, 85 degrees, or 90 degrees. In a preferred embodiment, the second slope range is from 60 degrees to 90 degrees. That is, the second slope range may be greater than or equal to 60 degrees and less than or equal to 90 degrees, so that the current position of the tip can be determined accurately.

The other tissue may be tissue other than the cortical bone, such as the cancellous bone, the soft tissue, or air. The interface between the cortical bone 21 and the other tissue represents that the cortical bone 21 is on the outer side of the other tissue, and an incision direction of the tip is from the cortical bone 21 towards the other tissue.

With reference to the first falling edges 42a and 42b in FIG. 3B, if the acoustic impedance signal is on the first falling edge and its average slope is within the second slope range, it may be accurately determined that the current position is the interface between the cortical bone 21 and the other tissue, thereby facilitating the operator 50 in knowing the current position of the ultrasonic osteotome 10.

In some embodiments, determining that the current position is the interface between the cortical bone and the other tissue in step S202 may include: determining that the current position is an interface between the cortical bone 21 and the cancellous bone 22 in response to determining that an acoustic impedance amplitude at an end point of the first falling edge is within the third preset range.

It can be understood that the acoustic impedance varies when the ultrasonic osteotome 10 is in different types of tissue. Therefore, an interface between the cortical bone 21 and specific tissue which the ultrasonic osteotome 10 is at may be determined through the acoustic impedance amplitude at the end point of the entire first falling edge 42a.

The third preset range is the acoustic impedance amplitude range of the ultrasonic osteotome 10 in the cancellous bone 22. By determining whether the acoustic impedance amplitude at the end point of the first falling edge 42a is within the third preset range, it may be accurately determined whether the current position is the interface between the cortical bone 21 and the cancellous bone 22.

In some embodiments, determining that the current position is the interface between the cortical bone and the other tissue in step S202 may further include: determining that the current position is an interface between the cortical bone and the soft tissue in response to determining that the acoustic impedance amplitude at the end point of the first falling edge is within the second preset range.

The second preset range is the acoustic impedance amplitude range of the ultrasonic osteotome 10 in the soft tissue. Similarly, by determining whether the acoustic impedance amplitude at the end point of the first falling edge 42b is within the second preset range, it may be accurately determined whether the current position is the interface between the cortical bone 21 and the soft tissue.

Certainly, determining that the current position is the interface between the cortical bone and the other tissue in step S202 may further include: determining that the current position is an interface between the cortical bone and air in response to determining that the acoustic impedance amplitude at the end point of the first falling edge is within the first preset range.

Still referring to FIG. 2, determining the current position of the ultrasonic osteotome 10 in the bone tissue 20 to be cut based on the acoustic impedance signal in step S102 may include the following step. Step S203: Determine that the current position is in the cortical bone in response to determining that the acoustic impedance signal is consistently within a fourth preset range throughout the preset time.

The fourth preset range may be an acoustic impedance amplitude range of the ultrasonic osteotome 10 in the cortical bone 21, which may have a minimum value. When the acoustic impedance amplitude is greater than the minimum value, it may be considered that the tip has been in the cortical bone 21. Similarly, by determining whether the acoustic impedance signal is within the fourth preset range, it may be accurately determined whether the current position is the cortical bone 21.

The preset time may be set to a plurality of sampling periods based on actual situations. By determining whether the amplitude of the acoustic impedance signal is consistently within the fourth preset range throughout the preset time, it may be determined whether the acoustic impedance signal is in a cortical bone impedance level section. As shown in FIG. 3B, the acoustic impedance signal is consistently within the fourth preset range throughout the preset time, that is, the acoustic impedance signal is in the cortical bone impedance level section 43b. The cortical bone impedance level section 43b indicates that the tip passes through the interface between the other tissue and the cortical bone 21 to be in the cortical bone 21. The length of the cortical bone impedance level section 43b on the horizontal coordinate axis may vary depending on the thickness of the cortical bone 21.

In addition, the fourth preset range may be different for cortical bone of different individuals or different positions of the same individual. For example, in FIG. 3B, the fourth preset range of the cortical bone impedance level 43b representing the outer cortical bone may be different from the fourth preset range of the cortical bone impedance level 43d representing the inner cortical bone.

In some embodiments, still referring to FIG. 2, determining the current position of the ultrasonic osteotome 10 in the bone tissue 20 to be cut based on the acoustic impedance signal in step S102 may include the following step. Step S204: Determine that the current position is in the cancellous bone in response to determining that the acoustic impedance signal is consistently within the third preset range throughout the preset time.

Since the hardness of the cortical bone is greater than that of the cancellous bone, the maximum value of the third preset range is less than the minimum value of the fourth preset range. The acoustic impedance amplitude in the cancellous bone should be 60% or less of the acoustic impedance amplitude in the cortical bone, that is, the average value of the third preset range is approximately 60% or less of the average value of the fourth preset range. The third preset range may be the acoustic impedance amplitude range of the ultrasonic osteotome 10 in the cancellous bone 22. As shown in FIG. 3B, when the acoustic impedance amplitude is within this range, it may be considered that the acoustic impedance signal is in a cancellous bone impedance level section 43c, and the tip is in the cancellous bone 22. The cancellous bone impedance level section 43c indicates that the tip passes through the interface between the other tissue such as the cortical bone 21 and the cancellous bone 22 to be in the cancellous bone 22. The length of the cancellous bone impedance level section 43c on the horizontal coordinate axis may vary depending on the thickness of the cancellous bone 22.

In this step S204, the method for determining that the current position is the cancellous bone is similar to the method for determining that the current position is the cortical bone in step S203. Refer to the above description of step S203. Details are not described again.

The above embodiment describes the method for determining the current position. The application of the present application will be described below with reference to exemplary types of bone tissue 20 to be cut.

FIG. 3C is a flowchart of a method for determining a position of an ultrasonic osteotome applied to the ultrasonic surgical system of FIG. 3A. Referring to FIG. 3A, FIG. 3B, and FIG. 3C, the method provided in this embodiment may be applied to surgery for cutting the three-layer bone tissue. For example, the surgery may be spinal fenestration surgery, etc. As shown in FIG. 3A, the bone tissue 20 to be cut includes the outer cortical bone 21a, the cancellous bone 22, and the inner cortical bone 21b arranged sequentially from outside to inside.

The method 300 for determining a position of an ultrasonic osteotome may include steps S301 to S303.

Step S301: Obtain an acoustic impedance signal of an ultrasonic osteotome 10.

Step S302: Determine a current position of the ultrasonic osteotome 10 in bone tissue 20 to be cut based on the acoustic impedance signal.

Step S303: Send first prompt information in response to determining that the current position is an interface between the inner cortical bone 21b and the soft tissue.

Steps S301 and S302 are the same as steps S101 and S102 in the above embodiment, and reference may be made to the description of the above embodiment.

It may be understood that FIG. 3B shows the acoustic impedance curve obtained when the three-layer bone tissue is cut through. The impedance level section 43a indicates that the tip of the ultrasonic osteotome 10 is consistently in air or the soft tissue. The first rising edge 41a indicates that the tip is at the interface between air or the soft tissue and the outer cortical bone 21a. The cortical bone impedance level section 43b indicates that the tip is in the outer cortical bone 21a. The first falling edge 42a indicates that the tip is at the interface between the outer cortical bone 21a and the cancellous bone 22. The cancellous bone impedance level section 43c indicates that the tip is in the cancellous bone 22. The first rising edge 41b indicates that the tip is at the interface between the cancellous bone 22 and the inner cortical bone 21b. The cortical bone impedance level section 43d indicates that the tip is in the inner cortical bone 21b. The first falling edge 42b indicates that the tip is at the interface between the inner cortical bone 21b and the soft tissue. An impedance level section 43e indicates that the tip has penetrated the structure of the three-layer bone tissue.

During the actual surgery, the inner side of the inner cortical bone 21b is usually the soft tissue such as the spinal cord or the brain. Once the ultrasonic osteotome 10 penetrates the inner cortical bone 21b, it will be prone to damaging nervous tissue.

Therefore, when it is determined that the current position is the interface between the inner cortical bone 21b and the soft tissue, if the operator 50 is a doctor, the first prompt information may be sent to the operator 50 in time, where the first prompt information may be a signal such as sound, light or vibration, to facilitate the doctor in stopping or lifting the ultrasonic osteotome 10. In addition, if the operator 50 is a surgical robot, the first prompt information sent may be directly used to control the ultrasonic osteotome 10 to stop cutting.

In conclusion, according to the above method 300, a current position of the tip may be detected, and a prompt may be provided for dangerous positions, thereby further improving the safety of surgery, and reducing accidental damage to the nervous tissue such as the spinal cord or the brain.

In some embodiments, determining that the current position is the interface between the inner cortical bone and the soft tissue in step S303 may include: determining that the current position is the interface between the inner cortical bone 21b and the soft tissue in response to determining that the current position is at the interface between the cortical bone 21 and the other tissue for the second time.

It may be understood that for the three-layer bone tissue, a cutting sequence is the outer cortical bone 21a, the cancellous bone 22, and the inner cortical bone 21b. The outer cortical bone 21a and the inner cortical bone 21b are both the cortical bone 21. For the method for determining the interface between the outer cortical bone or the inner cortical bone and the other tissue, refer to the method for determining the interface between the cortical bone and the other tissue in the above embodiment. In this embodiment, the number of times the current position is at the interface between the cortical bone 21 and the other tissue may be recorded to determine whether the current position is the interface between the outer cortical bone 21a and the soft tissue or the interface between the inner cortical bone 21b and the soft tissue. When the current position of the tip is at the interface between the cortical bone 21 and the other tissue for the first time, the interface is the interface between the outer cortical bone 21a and the cancellous bone 22. When the current position of the tip is at the interface between the cortical bone 21 and the other tissue for the second time, the interface is the interface between the inner cortical bone 21b and the soft tissue, so that it may be accurately determined whether the tip is in contact with the outer cortical bone 21a or the inner cortical bone 21b.

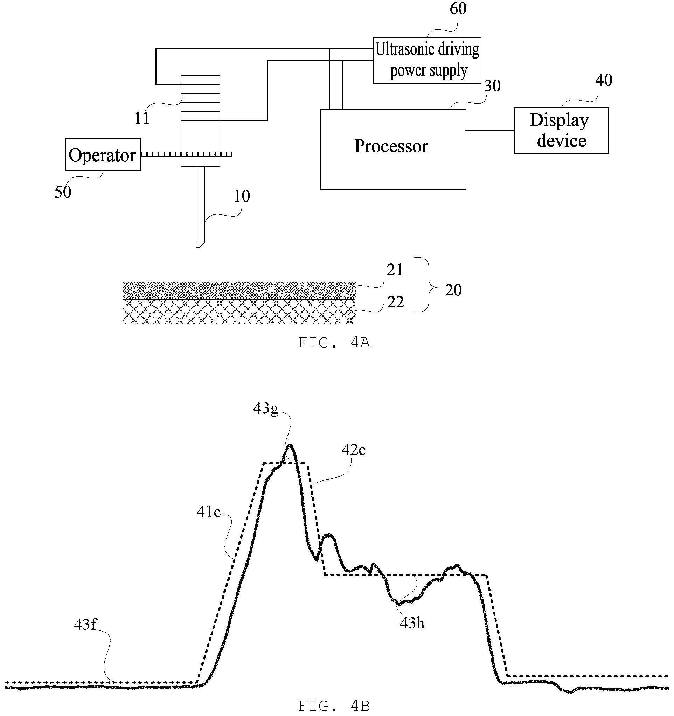

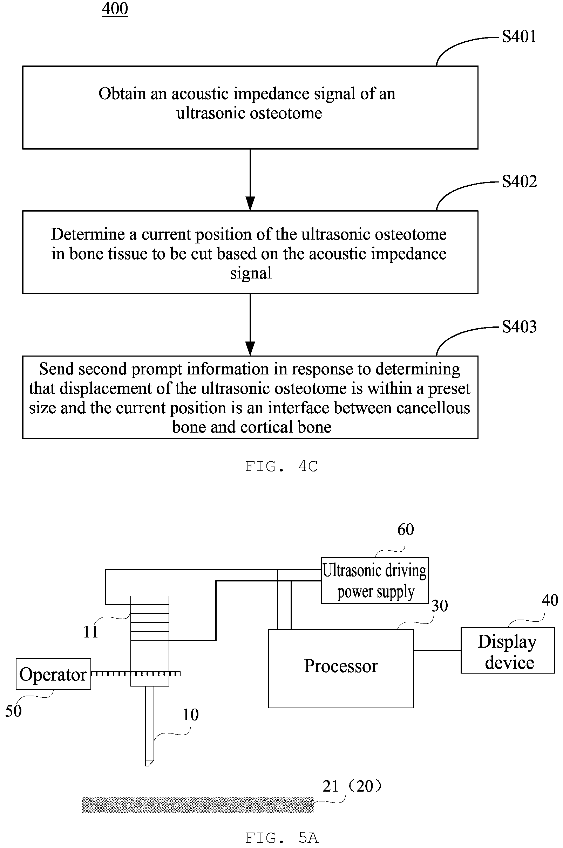

FIG. 4A is a diagram of a working principle of an ultrasonic surgical system according to some embodiments of the present application. FIG. 4B is a graph of acoustic impedance variations of an ultrasonic osteotome in FIG. 4A. FIG. 4C is a flowchart of a method for determining a position of an ultrasonic osteotome applied to the ultrasonic surgical system of FIG. 4A.

Referring to FIG. 4A, FIG. 4B, and FIG. 4C, this embodiment further provides a method 400 for determining a position of an ultrasonic osteotome, which may be applied to surgery for cutting a structure of two-layer bone tissue, such as pedicle screw drilling surgery. As shown in FIG. 4A, bone tissue 20 to be cut may include cortical bone 21 and cancellous bone 22 arranged sequentially from outside to inside, where the cortical bone 21 is on the outer side of the cancellous bone 22. The method may include steps S401 to S403.

Step S401: Obtain an acoustic impedance signal of an ultrasonic osteotome 10.

Step S402: Determine a current position of the ultrasonic osteotome 10 in the bone tissue 20 to be cut based on the acoustic impedance signal.

Step S403: Send second prompt information in response to determining that displacement of the ultrasonic osteotome 10 is within a preset size and the current position is an interface between the cancellous bone 22 and the cortical bone 21.

Steps S401 and S402 are the same as steps S101 and S102 in the above embodiment, and reference may be made to the description of the above embodiment.

It may be understood that FIG. 4B shows an acoustic impedance curve obtained when the two-layer bone tissue is cut through. An impedance level section 43f indicates that a tip of the ultrasonic osteotome 10 is consistently in air or soft tissue. A first rising edge 41c indicates that the tip is at an interface between air or the soft tissue and the cortical bone 21. A cortical bone impedance level section 43g indicates that the tip is in the cortical bone 21. A first falling edge 42c indicates that the tip is at the interface between outer cortical bone 21 and the cancellous bone 22. A cancellous bone impedance level section 43h indicates that the tip is in the cancellous bone 22.

In terms of the pedicle screw drilling surgery, during the actual surgery, drilling should penetrate the cortical bone 21 and stop in the cancellous bone 22. It may be understood that during this surgery, there is no phenomenon where the tip is at the interface between the cancellous bone and the cortical bone.

In step S403, the preset size may be a required drilling length, that is, the displacement of the ultrasonic osteotome 10 should reach the preset size. In addition, when the displacement of the ultrasonic osteotome 10 does not reach the preset size, and the current position is at the interface between the cancellous bone 22 and the cortical bone, it indicates that a cutting direction of the tip, i.e., a drilling direction, is wrong, and the ultrasonic osteotome 10 has been in contact with other cortical bone. In this case, the drilling direction needs to be adjusted. For the method for determining that the current position is the interface between the cancellous bone 22 and the cortical bone, refer to the above embodiment.

In addition, when the displacement of the ultrasonic osteotome 10 is within the preset size, and the current position is the interface between the cancellous bone 22 and the cortical bone 21, if the operator 50 is a doctor, the second prompt information may be sent to the operator 50 in time, where the second prompt information may be a signal such as sound, light or vibration, to prompt the doctor to adjust the cutting direction of the tip. In addition, if the operator 50 is a surgical robot, the second prompt information sent may be directly used to control the ultrasonic osteotome 10 to adjust the direction of the tip.

In conclusion, according to the above method 400, the current position of the tip may be detected, and the operator 50 may be prompted in time when the drilling direction is wrong, so that a drilling position is accurate, and the safety of surgery is further improved.

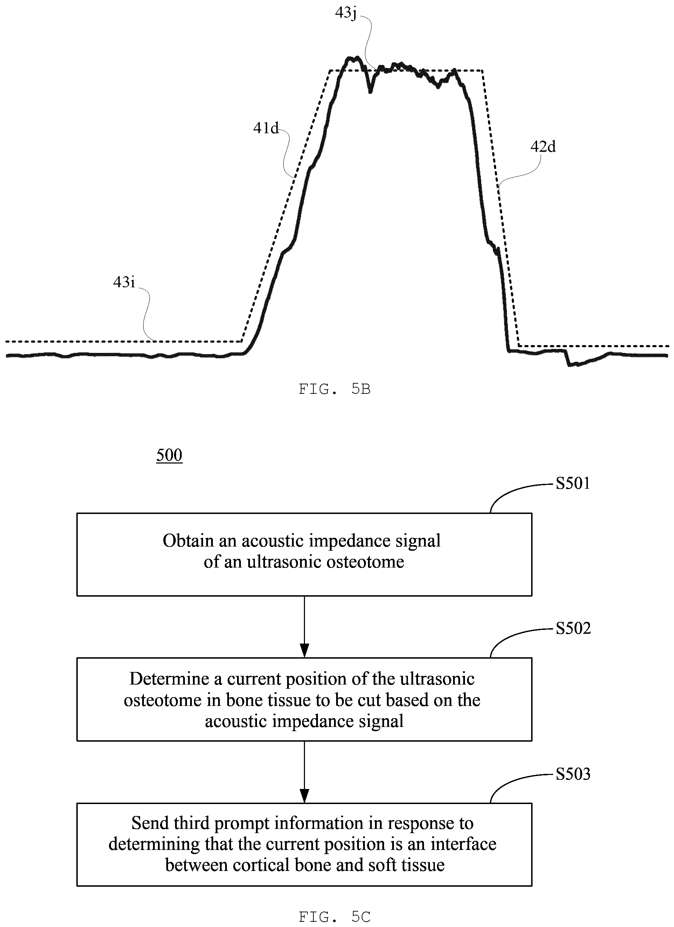

FIG. 5A is a diagram of a working principle of an ultrasonic surgical system according to some embodiments of the present application. FIG. 5B is a graph of acoustic impedance variations of an ultrasonic osteotome in FIG. 5A. FIG. 5C is a flowchart of a method for determining a position of an ultrasonic osteotome applied to the ultrasonic surgical system of FIG. 5A.

Referring to FIG. 5A, FIG. 5B, and FIG. 5C, this embodiment further provides a method 500 for determining a position of an ultrasonic osteotome, which may be applied to surgery for cutting single-layer cortical bone, such as a leg bone. As shown in FIG. 5A, bone tissue 20 to be cut may include cortical bone 21. The method may include steps S501 to S503.

Step S501: Obtain an acoustic impedance signal of an ultrasonic osteotome 10.

Step S502: Determine a current position of the ultrasonic osteotome 10 in bone tissue 20 to be cut based on the acoustic impedance signal.

Step S503: Send third prompt information in response to determining that the current position is an interface between the cortical bone and soft tissue.

Steps S501 and S502 are the same as steps S101 and S102 in the above embodiment, and reference may be made to the description of the above embodiment.

It may be understood that FIG. 5B shows an acoustic impedance curve obtained when the single-layer cortical bone is cut through. An impedance level section 43i indicates that a tip of the ultrasonic osteotome 10 is consistently in air or the soft tissue. A first rising edge 41d indicates that the tip is at the interface between air or the soft tissue and the cortical bone 21. A cortical bone impedance level section 43j indicates that the tip is in the cortical bone 21. A first falling edge 42d indicates that the tip is at the interface between the cortical bone 21 and the soft tissue.

In step S503, when the interface between the cortical bone 21 and the soft tissue appears, it is considered that the ultrasonic osteotome 10 has cut through the bone tissue. In this case, if the operator 50 is a doctor, the third prompt information may be sent to the operator 50 in time, where the third prompt information may be a signal such as sound, light or vibration, to facilitate the doctor in stopping or lifting the ultrasonic osteotome 10. In addition, if the operator 50 is a surgical robot, the third prompt information sent may be directly used to control the ultrasonic osteotome 10 to stop cutting.

For the method for determining that the current position is the interface between the cortical bone and the soft tissue, refer to the description of the above embodiment.

In conclusion, according to the above method 500, a current position of the tip may be detected, and a prompt may be provided in time after the ultrasonic osteotome 10 cuts through the bone tissue, thereby further improving the safety of surgery, and reducing damage to the soft tissue.

As shown in FIG. 1B, the present application further provides an ultrasonic surgical system, including: an ultrasonic osteotome 10 and a processor 30. The ultrasonic osteotome 10 is configured to cut bone tissue 20 to be cut. The processor 30 is configured to perform the method for determining a position of an ultrasonic osteotome described in the above embodiment.

The ultrasonic osteotome 10 may be controlled by a doctor or a surgical robot, and the processor 30 is a common structure capable of implementing data processing. The processor may acquire an acoustic impedance signal of a tip by means of a transducer 11, and process the acoustic impedance signal to determine a current position of the tip. The method for determining a position of an ultrasonic osteotome is the same as that in the above embodiment, and reference may be made to the above embodiment.

According to the ultrasonic surgical system provided in this embodiment, a current position of the ultrasonic osteotome 10 in the bone tissue to be cut is determined based on the acoustic impedance signal, so that an operator 50 can conveniently obtain the position of the ultrasonic osteotome 10 in real time. This has higher precision compared with image positioning, which can effectively prevent a movement path of the ultrasonic osteotome from deviating from a target path, thereby reducing damage to soft tissue during surgery and improving surgical efficiency.

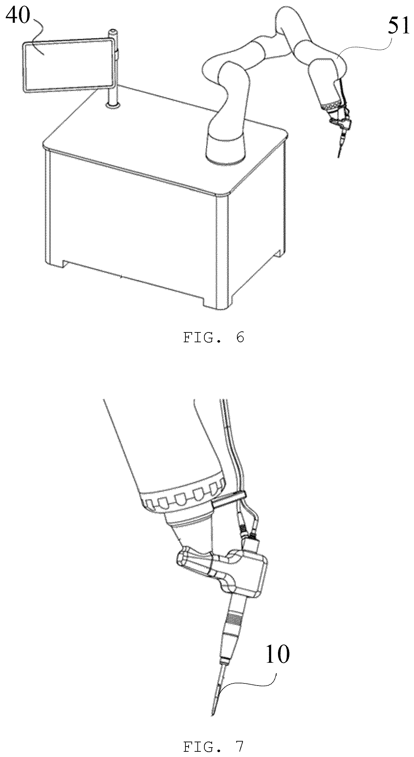

FIG. 6 is a diagram of a structure of an ultrasonic surgical system according to some embodiments of the present application; and FIG. 7 is a partial enlarged view of an ultrasonic osteotome in FIG. 6. Referring to FIG. 6 and FIG. 7, in some embodiments, the ultrasonic surgical system further includes: a surgical robot 51 and a display device 40. The surgical robot 51 is connected to the ultrasonic osteotome 10. The display device 40 is configured to display the acoustic impedance signal of the ultrasonic osteotome 10. The processor 30 is further configured to operate the ultrasonic osteotome 10 by controlling the surgical robot 51.

The surgical robot 51 may be a common robot capable of implementing motion control. The display device 40 may be a display structure such as a display, which may be configured to display acoustic impedance information and data feedback in real time, and may further be configured to display prompt information, etc.

The surgical robot 51 may be connected to the ultrasonic osteotome 10, and the processor 30 may be communicatively connected to the surgical robot 51, to send a control instruction to the surgical robot 51, thereby driving the ultrasonic osteotome 10 to move along the target path.

Certainly, the ultrasonic surgical system may further be provided with an image device, which may be configured to capture an image of the ultrasonic osteotome 10 and the bone tissue 20 to be cut. The display device 40 may display the image, to assist the operator 50 in intuitively knowing the position of the tip.

According to an embodiment of the present application, there are further provided a non-transitory computer-readable storage medium storing computer instructions and a computer program product.

The computer instructions are used to cause a computer to perform the method described in any of the above. The computer program product includes a computer program, where the computer program, when executed by a processor, causes the method described in any of the above to be implemented.

The computer may be any machine configured to perform processing and/or computing, which may be, but is not limited to, a workstation, a server, a desktop computer, a laptop computer, a tablet computer, a personal digital assistant, a smart phone, an in-vehicle computer, a wearable device, or any combination thereof. According to some implementations, the method for determining a position of an ultrasonic osteotome described above may also be implemented, in whole or at least in part, by the computer or a similar device or system.

The computer may include elements in connection with a bus or in communication with a bus (possibly via one or more interfaces). For example, the computer may include the bus, one or more processors, one or more input devices, and one or more output devices. The one or more processors may be any type of processors, and may include, but are not limited to, one or more general-purpose processors and/or one or more special processors (for example, special processing chips). The input device may be any type of device capable of inputting information to the computer, and may include, but is not limited to, a sensor (for example, the sensor for acquiring an image as described above), a mouse, a keyboard, a touch screen, a microphone and/or a remote controller. The output device may be any type of device capable of presenting information, and may include, but is not limited to, a display, a speaker (for example, the output device that may be used to output the sound data as described above), a video/audio output terminal, a vibrator and/or a printer. The computer may further include a non-transitory storage device or be connected to a non-transitory storage device. The non-transitory storage device (which may be, for example, used for implementing the computer readable storage medium as described above) may be non-transitory and may be any storage device capable of implementing data storage, and may include, but is not limited to, a disk drive, an optical storage device, a solid-state memory, a floppy disk, a flexible disk, a hard disk, a magnetic tape, or any other magnetic medium, an optical disk or any other optical medium, a read-only memory (ROM), a random access memory (RAM), a cache memory and/or any other memory chip or cartridge, and/or any other medium from which a computer can read data, instructions and/or code. The non-transitory storage device may be detached from an interface. The non-transitory storage device may have data/programs (including instructions)/code for implementing the foregoing methods and steps. The computer may further include a communication device. The communication device may be any type of device or system that enables communication with an external device and/or network, and may include, but is not limited to, a modem, a network card, an infrared communication device, a wireless communication device and/or a chipset, for example, a Bluetooth device, a 1302.11 device, a Wi-Fi device, a WiMax device, a cellular communication device and/or the like.

The computer may further include a working memory (which may be used to implement the memory of the foregoing system), which may be any type of working memory capable of storing programs (including instructions) and/or data useful to the working of the processor, and may include, but is not limited to, a random access memory and/or a read-only memory.

Software elements (programs) may be located in the working memory, and may include, but are not limited to, an operating system, one or more application programs, drivers, and/or other data and code. The instructions for executing the methods and steps may be included in the one or more applications. Executable code or source code of the instructions of the software elements (programs) may be stored in a non-transitory computer-readable storage medium (for example, the foregoing storage device), and may be stored in the working memory when executed (may be compiled and/or installed). The executable code or source code of the instructions of the software elements (programs) may alternatively be downloaded from a remote location.

Program code used to implement the method of the present application can be written in any combination of one or more programming languages. The program code may be provided for a processor or a controller of a general-purpose computer, a special-purpose computer, or other programmable data processing apparatuses, such that when the program code is executed by the processor or the controller, the functions/operations specified in the flowcharts and/or block diagrams are implemented. The program code may be completely executed on a machine, or partially executed on a machine, or may be, as an independent software package, partially executed on a machine and partially executed on a remote machine, or completely executed on a remote machine or a server.

It should be understood that steps may be reordered, added, or deleted based on the various forms of procedures shown above. For example, the steps recorded in the present application may be performed in parallel, in order, or in a different order, provided that the desired result of the technical solutions in the present application can be achieved, which is not limited herein.

It should be understood that, in this description, the orientations or positional relationships or dimensions denoted by the terms, such as "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial" and "circumferential", are the orientations or positional relationships or dimensions shown on the basis of the drawings, and these terms are used merely for ease of description, rather than indicating or implying that the device or element referred to must have particular orientations and be constructed and operated in the particular orientations, and therefore should not be construed as limiting the scope of protection of the present application.

In addition, the terms such as "first", "second" and "third" are merely for descriptive purposes and should not be construed as indicating or implying relative importance or implicitly indicating the number of technical features indicated. Thus, the features defined with "first", "second" and "third" may explicitly or implicitly include one or more features. In the description of the present application, the term "plurality of" means two or more, unless specifically and specifically limited otherwise.

In the present application, unless expressly stated or limited otherwise, the terms such as "mounting", "connection", "connected" and "fixing" should be interpreted broadly, for example, either fixed or detachable connection, or integration; may be a mechanical connection, or an electrical connection, or communication; and may be a direct connection or an indirect connection by means of an intermediate medium, or may be internal communication between two elements or interaction between the two elements. For those of ordinary skills in the art, the specific meaning of the terms mentioned above in the present application may be construed according to specific circumstances.

In the present application, unless expressly stated or limited otherwise, the expression of the first feature being "above" or "below" the second feature may include the case that the first feature is in direct contact with the second feature, and may also include the case that the first and second features are not in direct contact but are contacted via another feature therebetween. Furthermore, the first feature being "over", "above" or "on" the second feature includes the case that the first feature is directly or obliquely above the second feature, or merely indicates that the first feature is at a higher level than the second feature. The first feature being "below", "under" or "beneath" the second feature includes the case that the first feature is directly or obliquely below the second feature, or merely indicates that the first feature is at a lower level than the second feature.

This description provides many different embodiments or examples that can be used to implement the present application. It should be understood that these various embodiments or examples are purely illustrative and are not intended to limit the scope of protection of the present application in any way. On the basis of the disclosure of the description of the present application, those skilled in the art will be able to conceive of various changes or substitutions. Any changes or substitutions shall fall within the scope of protection of the present application. Therefore, the scope of protection of the present application shall be subject to the scope of protection of the claims.

## Claims

1. A method for determining a position of an ultrasonic osteotome, comprising:
obtaining an acoustic impedance signal of the ultrasonic osteotome; and
determining a current position of the ultrasonic osteotome in bone tissue to be cut based on the acoustic impedance signal.

2. The method according to claim 1, wherein the determining a current position of the ultrasonic osteotome in bone tissue to be cut based on the acoustic impedance signal comprises:
determining that the current position is an interface between other tissue and cortical bone in response to determining that the acoustic impedance signal is on a first rising edge in a preset time and an average slope of the first rising edge is within a first slope range.

3. The method according to claim 2, wherein the determining that the current position is an interface between other tissue and cortical bone comprises:
determining that the current position is an interface between air and the cortical bone in response to determining that an acoustic impedance amplitude at a start point of the first rising edge is within a first preset range.

4. The method according to claim 2, wherein the determining that the current position is an interface between other tissue and cortical bone comprises:
determining that the current position is an interface between soft tissue and the cortical bone in response to determining that an acoustic impedance amplitude at a start point of the first rising edge is within a second preset range.

5. The method according to claim 2, wherein the determining that the current position is an interface between other tissue and cortical bone comprises:
determining that the current position is an interface between cancellous bone and the cortical bone in response to determining that an acoustic impedance amplitude at a start point of the first rising edge is within a third preset range.

6. The method according to claim 1, wherein the determining a current position of the ultrasonic osteotome in bone tissue to be cut based on the acoustic impedance signal comprises:
determining that the current position is an interface between cortical bone and other tissue in response to determining that the acoustic impedance signal is on a first falling edge in a preset time and an average slope of the first falling edge is within a second slope range.

7. The method according to claim 6, wherein the determining that the current position is an interface between cortical bone and other tissue comprises:
determining that the current position is an interface between the cortical bone and cancellous bone in response to determining that an acoustic impedance amplitude at an end point of the first falling edge is within a third preset range.

8. The method according to claim 6, wherein the determining that the current position is an interface between cortical bone and other tissue comprises:
determining that the current position is an interface between the cortical bone and soft tissue in response to determining that an acoustic impedance amplitude at an end point of the first falling edge is within a second preset range.

9. The method according to claim 1, wherein the determining a current position of the ultrasonic osteotome in bone tissue to be cut based on the acoustic impedance signal comprises:
determining that the current position is in cortical bone in response to determining that the acoustic impedance signal is within a fourth preset range throughout a preset time.

10. The method according to claim 1, wherein the determining a current position of the ultrasonic osteotome in bone tissue to be cut based on the acoustic impedance signal comprises:
determining that the current position is in cancellous bone in response to determining that the acoustic impedance signal is consistently within a third preset range throughout a preset time.

11. The method according to any one of claims 1 to 10, wherein the bone tissue to be cut comprises outer cortical bone, the cancellous bone, and inner cortical bone arranged sequentially from outside to inside; and the method further comprises: sending first prompt information in response to determining that the current position is an interface between the inner cortical bone and the soft tissue.

12. The method according to claim 11, wherein the determining that the current position is an interface between the inner cortical bone and the soft tissue comprises:
determining that the current position is the interface between the inner cortical bone and the soft tissue in response to determining that the current position is at the interface between the cortical bone and the other tissue for the second time.

13. The method according to any one of claims 1 to 10, wherein the bone tissue to be cut comprises the cortical bone and the cancellous bone arranged sequentially from outside to inside; and the method further comprises:
sending second prompt information in response to determining that displacement of the ultrasonic osteotome is within a preset size and the current position is the interface between the cancellous bone and the cortical bone.

14. The method according to any one of claims 1 to 10, wherein the bone tissue to be cut comprises the cortical bone; and the method further comprises:
sending third prompt information in response to determining that the current position is the interface between the cortical bone and the soft tissue.

15. An ultrasonic surgical system, comprising:
an ultrasonic osteotome configured to cut bone tissue to be cut; and
a processor configured to perform the method according to any one of claims 1 to 14.

16. The system according to claim 15, wherein the system further comprises:
a surgical robot connected to the ultrasonic osteotome; and
a display device configured to display an acoustic impedance signal of the ultrasonic osteotome; and
the processor is further configured to operate the ultrasonic osteotome by controlling the surgical robot.

17. A non-transitory computer-readable storage medium storing computer instructions, wherein the computer instructions are used to cause the computer to perform the method according to any one of claims 1 to 14.

18. A computer program product, comprising a computer program, wherein the computer program, when executed by a processor, causes the method according to any one of claims 1 to 14 to be implemented.
